(19) Europäisches Patentamt
European Patent Office
Office européen des brevets

(11) Veröffentlichungsnummer: **0 120 438**
**A1**

# (12) EUROPÄISCHE PATENTANMELDUNG

(21) Anmeldenummer: **84103021.6**

(22) Anmeldetag: **20.03.84**

(51) Int. Cl.³: **C 07 D 217/04**
A 61 K 31/47

(30) Priorität: **25.03.83 DE 3310878**

(43) Veröffentlichungstag der Anmeldung:
**03.10.84 Patentblatt 84/40**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Schmitt, Karl, Dr.**
**Hasenpfad 3**
**D-6232 Bad Soden am Taunus(DE)**

(72) Erfinder: **Kruse, Hansjörg, Dr.**
**Feldbergstrasse 70**
**D-6233 Kelkheim (Taunus)(DE)**

(72) Erfinder: **Schacht, Ulrich, Dr.**
**Finkenweg 12**
**D-6238 Hofheim am Taunus(DE)**

(72) Erfinder: **Kunstmann, Rudolf, Dr.**
**Auf der Ahl 37**
**D-6200 Wiesbaden(DE)**

(54) **Optische Antipoden von 8-Amino-2-methyl-4-phenyl-1,2,3,4-tetrahydroisochinolin, Verfahren zu ihrer Herstellung und sie enthaltende Arzneimittel mit antidepressiver Wirkung.**

(57) Es werden die beiden optischen Antipoden (+)- und (–) -8- Amino -2- methyl -4- phenyl-1, 2, 3, 4- tetrahydro-isochinolin beschrieben sowie Verfahren zu ihrer Herstellung aus racemischem 8- Amino -2- methyl -4- phenyl-1, 2, 3, 4-tetrahydro-isochinolin mit einer optisch aktiven Säure.

Die beiden Antipoden haben antidepressive Wirkungen und eignen sich daher zur Behandlung von Depressionen.

EP 0 120 438 A1

Optische Antipoden von 8-Amino-2-methyl-4-phenyl-1,2,3,4-tetrahydroisochinolin, Verfahren zu ihrer Herstellung und sie enthaltende Arzneimittel mit antidepressiver Wirkung.

Gegenstand der Erfindung sind die optischen Antipoden von 8-Amino-2-methyl-4-phenyl-1,2,3,4-tetrahydroisochinolin (Nomifensin) der Formel I

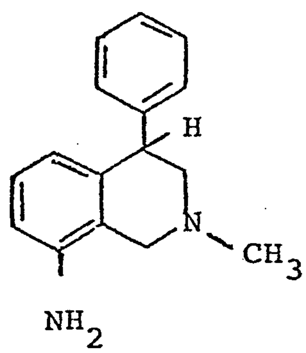

(I)

und deren Salze mit physiologisch verträglichen Säuren.

Gegenstand der Erfindung ist ebenso ein Verfahren zur Herstellung der beiden optischen Antipoden, das dadurch gekennzeichnet ist, daß man racemisches Nomifensin mit einer optisch aktiven Säure zu einem schwerlöslichen diastereomeren Salz umsetzt, anschließend aus dem Salz ein reines Enantiomeres und aus der verbliebenen Mutterlauge das zweite Enantiomere abscheidet und isoliert und gegebenenfalls die Enantiomeren in Salze mit einer physiologisch verträglichen Säure überführt.

Nach der deutschen Patentschrift 17 95 829 (US-Patentschrift 3 577 424) ist Nomifensin als Antidepressivum bekannt. Da Nomifensin in der 4-Position des Isochinolinringes ein asymmetrisches Kohlenstoffatom aufweist, kann es in zwei optische Antipoden gespalten werden. Dabei war eine Verdopplung der Wirkungsstärke eines Enantiomeren pro Gewichtseinheit und damit bei gleicher Wirkung eine Halbierung seiner notwendigen Wirkdosis zu erwarten. Bei der biologischen Prüfung zeigte sich jedoch überraschenderweise, daß das (+)-Enantiomere an verschiedenen Tiermodellen um den Faktor 3 - 4 stärker wirksam war als das Racemat, z.B. bei der Prüfung der spontanen motorischen Aktivität und der antagonistischen Wirkung bei der durch Oxotremorin hervorgerufenen Hypothermie. Andererseits zeigte auch das (-)-Enantiomere in unerwarteter Weise eine biologische Wirkung an anderen pharmakologischen Modellen, z.B. bei der Prüfung der antagonistischen Wirkung bei der durch Reserpin hervorgerufenen Hypothermie.

Beide Antipoden des Nomifensins besitzen demnach eine biologische Wirkung, die den Einsatz eines jeden Antipoden als Antidepressivum erlaubt, wobei der Wirkmechanismus überraschenderweise von unterschiedlicher Natur ist.

Zur Trennung des Racemats von Nomifensin der Formel I in die optischen Antipoden wird zweckmäßig eine Lösung von racemischem Nomifensin in einem Alkohol, wie Methanol, Ethanol oder Isopropanol oder in einem Keton, wie Aceton, mit der Lösung einer optisch aktiven Säure, beispielsweise (D)-(-) oder (L)-(+)-Weinsäure oder O,O'-Ditolyl-(D)-(-) oder O,O'-Ditolyl-(L)-(+)-Weinsäure oder vorteilhaft N-Benzolsulfonyl-(L)-(+)-glutaminsäure in einem der oben genannten Alkohole oder Ketone versetzt und bei Raumtemperatur das diastereomere Salz auskristallisieren lassen.

Am besten hat sich die Umsetzung des Racemats der Formel I mit N-Benzolsulfonyl-(L)-(+)-glutaminsäure in Ethanol beim Einsatz von äquimolaren Mengen bewährt. Aus dem abgetrennten Niederschlag, der in diesem Fall praktisch zu 100 % aus dem Salz mit dem (+)-Enantiomeren von I besteht, wird durch Zugabe einer starken Base wie Natronlauge in Wasser die zugrunde liegende optisch aktive Base freigesetzt und mit einem organischen Lösungsmittel wie Methylenchlorid extrahiert. Nach dem Trocknen und Einengen der organischen Phase hinterbleibt die praktisch reine (+)-Base der Formel I.

Zur Gewinnung des (-)-Enantiomeren engt man die Mutterlauge der ersten Kristallisation ein, setzt nach Zugabe von Wasser die optisch aktiven Base mit einer starken Base wie Natronlauge frei, extrahiert mit dem organischen Lösungsmittel, trocknet und engt ein.

Die so gewonnenen (+)- und (-)-Basen können durch Zugabe einer physiologisch verträglichen Säure, zweckmäßig in Ethanol oder Aceton, in das gewünschte Salz übergeführt werden.

Jeder der beiden optischen Antipoden des Nomifensins hat wertvolle pharmakologische Eigenschaften, die sich in Tiermodellen zeigen, die erfahrungsgemäß die Vorhersage einer antidepressiven Wirkung in der Klinik erlauben. Beide Antipoden eignen sich deshalb als Arzneimittel zur Therapie von Depressionen.

Die beiden optischen Antipoden der Formel I können entweder für sich allein oder zweckmäßig mit physiologisch verträglichen Hilfs- und/oder Trägerstoffen vermischt als Arzneimittel verwendet werden. Für eine orale Anwendungsform wird ein Antipode der Formel I mit den dafür üblichen Substanzen vermischt und durch übliche Methoden in geeignete Darreichungsformen gebracht, wie Tabletten, Steckkapseln, wäßrige oder alkoholische Suspensionen oder wäßrige oder alkoholische Lösungen. Als inerte Träger für feste Applikationsformen können z.B. Magnesiumcarbonat, Milchzucker oder Maisstärke verwendet werden.

Zur intravenösen Applikation kann jeder der beiden Antipoden oder eines seiner Salze mit einer physiologisch verträglichen organischen oder anorganischen Säure in dafür üblichen Lösungsmitteln gelöst werden. Als organische Säuren seien beispielsweise genannt Essigsäure, Propionsäure, Milchsäure, Malonsäure, Maleinsäure, Fumarsäure, Zitronensäure, Apfelsäure, Weinsäure, Benzoesäure, Oxethansulfonsäure, Acetursäure (Acetylaminoessigsäure), Ethylendiamintetraessigsäure oder Ambonsäure (1,1'-Methylen-bis-(2-hydroxy-3-naphthoesäure). Als organische Säuren kommen beispielsweise in Betracht Halogenwasserstoffsäure, wie Chlorwasserstoffsäure oder Bromwasserstoffsäure, Schwefelsäure, Phosphorsäure und Amidosulfonsäure.

Als Lösungsmittel für die intravenöse Applikation kommen in Betracht Wasser oder physiologische Kochsalzlösung, die mit weiteren Zusätzen wie Alkoholen (z.B. Propandiol, Glycerin) oder Zuckerlösungen (z.B. Glukose- oder Mannitlösungen) versehen sein können.

Experimenteller Teil

Beispiel 1

(+)-8-Amino-2-methyl-4-phenyl-1,2,3,4-tetrahydroisochinolin

0,6 Mol (±)-8-Amino-2-methyl-4-phenyl-1,2,3,4-tetrahydroisochinolin werden mit 0,6 Mol N-Benzolsulfonyl-(L)-(+)-glutaminsäure in 5 l Ethanol versetzt und bis zur beendeten Kristallisation bei Raumtemperatur gehalten. Der Kristallbrei wird abgesaugt mit wäßriger 2n Natronlauge versetzt, und die freigesetzte Base in Methylenchlorid aufgenommen. Nach dem Trocknen der Lösung mit Magnesiumsulfat engt man ein und überführt die rohe Base in Ethanol durch Zugabe von äquimolaren Mengen der gewünschten Säure in das entsprechende Salz.

Man erhält so:

N-Benzolsulfonyl-(L)-(+)-glutamat Fp. 201 - 204°C (Z.)

Freie Base Fp. 82 - 84°C $[\alpha]_D^{20}$ = +78,1°C (c=0,5; $CH_3OH$)

Hydrochlorid Fp. 233 - 236°C (Z.) $[\alpha]_D^{20}$ = +54.9°C (c= 0,5; $H_2O$)

Maleinat Fp. 183 - 185°C (Z.) $[\alpha]_D^{20}$ = +34,0°C (c= 0,5; $CH_3OH$)

Beispiel 2

(-)-8-Amino-2-methyl-4-phenyl-1,2,3,4-tetrahydroisochinolin

Die nach der Kristallisation von (+)-8-Amino-2-methyl-4-phenyl-1,2,3,4-tetrahydroisochinolin mit Benzolsulfonyl-(L)-(+)-glutaminsäure (s. Beispiel 1) erhaltene Mutterlauge wird eingeengt, das so erhaltene rohe Salz in Wasser ausgeschlämmt

und mit 2n NaOH versetzt. Die freigesetzte Base nimmt man in Methylenchlorid auf, trocknet, engt ein und überführt die so erhaltene Rohbase in Ethanol durch Zugabe der gewünschten Säure in äquimolaren Mengen in das entsprechende Salz.

Man erhält so:

| | | | |
|---|---|---|---|
| Hydrochlorid | Fp. 232 - 235°C (Z.) | $[\alpha]_D^{20}$=-54,8°C | (c= 0,5; $H_2O$) |
| Maleinat | Fp. 184 - 185°C (Z.) | $[\alpha]_D^{20}$ =-35,0°C | (c= 0,5; $CH_3OH$) |

Patentansprüche:

1. (+)-8-Amino-2-methyl-4-phenyl-1,2,3,4-tetrahydro-isochinolin.

2. (-)-8-Amino-2-methyl-4-phenyl-1,2,3,4-tetrahydro-isochinolin.

3. Verfahren zur Herstellung der beiden optischen Antipoden nach den Ansprüchen 1 und 2, dadurch gekennzeichnet, daß man racemisches 8-Amino-2-methyl-4-phenyl-1,2,3,4-tetrahydro-isochinolin mit einer optisch aktiven Säure zu einem schwerlöslichen diastereomeren Salz umsetzt, anschließend aus dem Salz ein reines Enantiomeres und aus der verbliebenen Mutterlauge das zweite Enantiomere abscheidet und isoliert und gegebenenfalls die Enantiomeren in Salze mit einer physiologisch verträglichen Säure überführt.

4. Arzneimittel, dadurch gekennzeichnet, daß es die optisch aktive Verbindung nach Anspruch 1 oder 2 oder ein Salz einer dieser Verbindungen mit einer physiologisch verträglichen Säure enthält oder daraus besteht.

5. Verwendung der optisch aktiven Verbindung nach Anspruch 1 oder 2 oder eines Salzes einer dieser Verbindungen mit einer physiologisch verträglichen Säure zur Behandlung von Depressionen.

PATENTANSPRUCH für Österreich:

Verfahren zur Herstellung der beiden optischen Antipoden (+)-8-Amino-2-methyl-4-phenyl-1,2,3,4-tetrahydro-isochinolin und (-)-8-Amino-2-methyl-4-phenyl-1,2,3,4-tetrahydro-isochinolin, dadurch gekennzeichnet, daß man racemisches 8-Amino-2-methyl-4-phenyl-1,2,3,4-tetrahydro-isochinolin mit einer optisch aktiven Säure zu einem schwerlöslichen diastereomeren Salz umsetzt, anschließend aus dem Salz ein reines Enantiomeres und aus der verbliebenen Mutterlauge das zweite Enantiomere abscheidet und isoliert und gegebenenfalls die Enantiomeren in Salze mit einer physiologisch verträglichen Säure überführt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0120438
Nummer der Anmeldung

EP 84103021.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE - B2 - 1 795 830 (HOECHST AG)<br>* Patentanspruch; Spalte 3, Zeilen 12-20 *<br>-- | 1,2,4 | C 07 D 217/04<br>A 61 K 31/47 |
| A,D | DE - B2 - 1 795 829 (HOECHST AG)<br>* Patentanspruch; Spalte 4, Zeilen 14-20 *<br>-- | 1,2,4 | |
| A | EP - B1 - 0 000 013 (HOECHST AG)<br>* Patentansprüche *<br>-- | 1,2,4 | |
| A | E.L. ELIEL "Stereochemie der Kohlenstoffverbindungen" 1966<br>VERLAG CHEMIE, GmbH Weinheim/ Bergstraße,<br>Seiten 64-65 *<br>---- | 3 | |

RECHERCHIERTE SACHGEBIETE (Int. Cl. 3)

C 07 D 217/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4
Unvollständig recherchierte Patentansprüche: -
Nicht recherchierte Patentansprüche: 5
Grund für die Beschränkung der Recherche:

Artikel 52(4) EPÜ Verfahren zur therapeutischen Behandlung des menschlichen oder tierischen Körpers

| Recherchenort | Abschlußdatum der Recherche | Prüfer |
|---|---|---|
| WIEN | 26-06-1984 | ONDER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03 82